# EUROPEAN PATENT APPLICATION

(11) **EP 3 144 837 A1**
(43) Date of publication of application: **22.03.2017**
(21) Application number: 15185243.1
(22) Date of filing: 15.09.2015
(51) Int. Cl.: G06F 21/34, G06F 3/0354, G06F 3/0488, G06F 19/00

(54) **A TECHNIQUE FOR AUTHENTICATION OF A TOUCH INPUT**

(71) Applicant: Siemens Aktiengesellschaft, 80333 München (DE)
(72) Inventor: Groten, Raphaela, 80469 München (DE); Johnson, Rebecca, 81825 München (DE)

(57) **Abstract**

A system and a method for authentication of an incoming touch-input received by a touch-sensitive surface are presented. The system includes a verification module and an authentication unit. The verification module receives information from the touch-sensitive surface corresponding to the incoming touch-input, and determines a received pattern of the incoming touch-input from the information. The verification module compares the received pattern to an authenticated pattern. The authentication unit includes an unique identity part configured to provide to the touch-sensitive surface a touch input as the incoming touch-input. The touch input provides a unique pattern as the received pattern when identified by the verification module. The unique pattern is same as the authenticated pattern. The verification module controls the touch-sensitive surface in such way that a region of the touch-sensitive surface activates the medical function only if the received pattern matches the authenticated pattern.

## Description

The present invention relates to techniques to control medical devices and more particularly to techniques to control medical devices via touch-based input.

In present times, medical technology has witnessed a spurt in numbers and types of medical devices. Medical centers, such as a diagnostic lab or a hospital use a variety of such medical devices. The manufacturers of such medical devices have been striving incessantly to make the use of these medical devices as user friendly as possible. With the advent of touch-screen technology, a lot of such medical devices have witnessed integration of touch-screens into the medical devices or into a control of the medical device to make it easy for an operator of such a medical device to provide inputs to the medical device to perform one or more functions.

However, the use of touch-based inputs has given rise to certain disadvantageous. Most of the touch-screen based medical devices lack a security or authentication process to ensure that the input received by the medical device by use of the touch-screen is an authentic input i.e. to ensure that the input was provided by an authorized operator and/or the input on the touch-screen was intended by such an operator to be a certain input. With ease of usability in providing touch-based input, operators of such medical devices providing the touch-based inputs to operate one or more function of the medical device are often less attentive and as a result end up providing a wrong input i.e. an input provided unintentionally by the operator because of lack of attention during providing of the touch-based input. Furthermore, any person including an unauthorized person is capable to provide touch-based inputs.

Thus the object of the present technique is to reduce the above mentioned disadvantages by providing a technique for authentication of an incoming touch-input received by a touch-sensitive surface that controls an operation of a medical function of a medical device. The technique is aimed at making the control of the operation of the medical function of the medical device more secure.

The above objects are achieved by a system for authentication of an incoming touch-input received by a touch-sensitive surface according to claim 1 of the present technique and a method for authentication of an incoming touch-input received by a touch-sensitive surface according to claim 12 of the present technique. Advantageous embodiments of the present technique are provided in dependent claims. Features of claim 1 may be combined with features of claims dependent on claim 1, and features of these dependent claims can be combined together. Similarly, features of claim 12 may be combined with features of claims dependent on claim 12, and features of these dependent claims can be combined together.

According to an aspect of the present technique a system for authentication of an incoming touch-input is presented. The incoming touch-input is received by a touch-sensitive surface. At least a region on the touch-sensitive surface is capable of activating a medical function of a medical device. The system includes a verification module and an authentication unit. The verification module receives an information from the touch-sensitive surface. The information corresponds to the incoming touch-input. The verification module then determines a received pattern of the incoming touch-input from the information. Thereafter, the verification module compares the received pattern to an authenticated pattern. The authentication unit includes an unique identity part. The unique identity part provides to the touch-sensitive surface a touch input as the incoming touch-input. The touch input provides a unique pattern as the received pattern when identified by the verification module. The unique pattern is same as the authenticated pattern. The verification module controls the touch-sensitive surface in such way that the region of the touch-sensitive surface activates the medical function only if the received pattern matches the authenticated pattern. As a result of the system of the present technique, possibility of activation of the medical function of the medical device by unauthenticated input is at least partially obviated. This in turn also ensures that only authorized personnel, possessing the authentication unit, can activate the medical function via the touch-sensitive surface. Thus operation of the medical function of the medical device is made more secure.

In an embodiment of the system, the verification module controls the touch-sensitive surface in such way that the region of the touch-sensitive surface activates the medical function only if the incoming touch-input is confined entirely in the region on the touch-sensitive surface. Thus accidental activation of the medical function is at least partially obviated, because an operator or authorized personnel providing the touch input via the authentication unit needs to pay more attention to the input provided via the touch-sensitive surface in order to achieve activation of the medical function. This ensures attentiveness of the operator or authorized personnel for operating medical devices.

In another embodiment, the system further includes a touch-screen unit having the touch-sensitive surface. Thus the system self sustained.

Furthermore, in another embodiment of the system, the touch-screen unit is an integral part of the medical device. Thus the system can be implemented by using a touch-surface of the medical device as the touch-screen unit. This makes integration of system possible with existing medical devices with touch-surfaces.

The touch-screen unit is remotely located with regard to the medical device in another embodiment of the system. Thus the system can be used to control operations of one or more functions of a medical device from a central location or central controller. Similarly, the system can be used to control operations of one or more functions of one or more medical device from the central location or the central controller.

In another embodiment of the system, the touch-screen unit communicates with the medical device via wireless communication. This provides a simple way of implementing the system.

In another embodiment of the system, the verification module and the touch-screen unit communicate with each other wirelessly. Thus a system can be implemented as distinct physical unit - the touch screen unit and the verification module - which provides greater flexibility in integrating the system with one or more medical devices the system is intended to control by activation of one or more medical functions of the one or more medical devices.

In another embodiment of the system, the touch-screen unit controls a C-arm based medical device via the touch-sensitive surface. Thus the system can be used to control movements and/or exposures provided to a subject via the C-arm based medical device more carefully.

In another embodiment of the system, the authentication unit includes an elongated body. The unique identity part is located at one end of the elongated body and is composed of a plurality of mechanical structures. These mechanical structures are positioned in a predefined relation to each other. The mechanical structures are simultaneously in contact with the touch-sensitive surface. This provides a simple design of the authentication unit and thus implementing the system becomes easier.

In another embodiment of the system the plurality of mechanical structures of the unique identity part are at least three protrusions extending outward from the one end of the elongated body. This provides a very basic and simple design of the authentication unit.

According to another aspect of the present technique a method for authentication of an incoming touch-input is presented. The incoming touch-input is received by a touch-sensitive surface. At least a region on the touch-sensitive surface is capable of activating a medical function of a medical device. First, an information from the touch-sensitive surface is received. The information so received corresponds to the incoming touch-input. Thereafter, a received pattern of the incoming touch-input from the information is determined. The received pattern is then compared to an authenticated pattern. Finally, the medical function is activated if and only if the received pattern matches the authenticated pattern.

In an embodiment of the method, the medical function is activated, if and only if the received pattern matches the authenticated pattern and if the incoming touch-input is entirely confined in the region on the touch-sensitive surface.

In another embodiment of the method, a processor receives the information from the touch-sensitive surface wirelessly. The processor determines the received pattern of the incoming touch-input from the information and compares the received pattern to an authenticated pattern.

In another embodiment of the method the authenticated pattern is stored in a memory module and the processor receives the authenticated pattern from the memory module.

Moreover, the system and the method, presented in the present technique, are especially advantageous in cases where the touch-sensitive surfaces are used to carry out or provide critical instructions to a medical device, for example carrying out an exposure of medical radiations to a subject.

The present technique is further described hereinafter with reference to illustrated embodiments shown in the accompanying drawing, in which:
- FIG 1: schematically illustrates an exemplary embodiment of a system of the present technique;
- FIG 2: schematically illustrates another exemplary embodiment of the system of FIG 1;
- FIG 3: schematically illustrates a function of the exemplary embodiment of the system;
- FIG 4: schematically illustrates another function of the exemplary embodiment of the system;
- FIG 5: schematically illustrates an exemplary embodiment of an authentication unit of the system;
- FIG 6: schematically illustrates yet another exemplary embodiment of the system of FIG 1;
- FIG 7: schematically illustrates another exemplary embodiment of the system of FIG 1 with use of the authentication unit in a first way;
- FIG 8: schematically illustrates another exemplary embodiment of the system of FIG 1 with use of the authentication unit in a second way; and
- FIG 9: depicts a flow chart representing an exemplary embodiment of a method of the present technique; in accordance with aspects of the present technique.

Hereinafter, above-mentioned and other features of the present technique are described in details. Various embodiments are described with reference to the drawing, wherein like reference numerals are used to refer to like elements throughout. In the following description, for purpose of explanation, numerous specific details are set forth in order to provide a thorough understanding of one or more embodiments. It may be noted that the illustrated embodiments are intended to explain, and not to limit the invention. It may be evident that such embodiments may be practiced without these specific details.

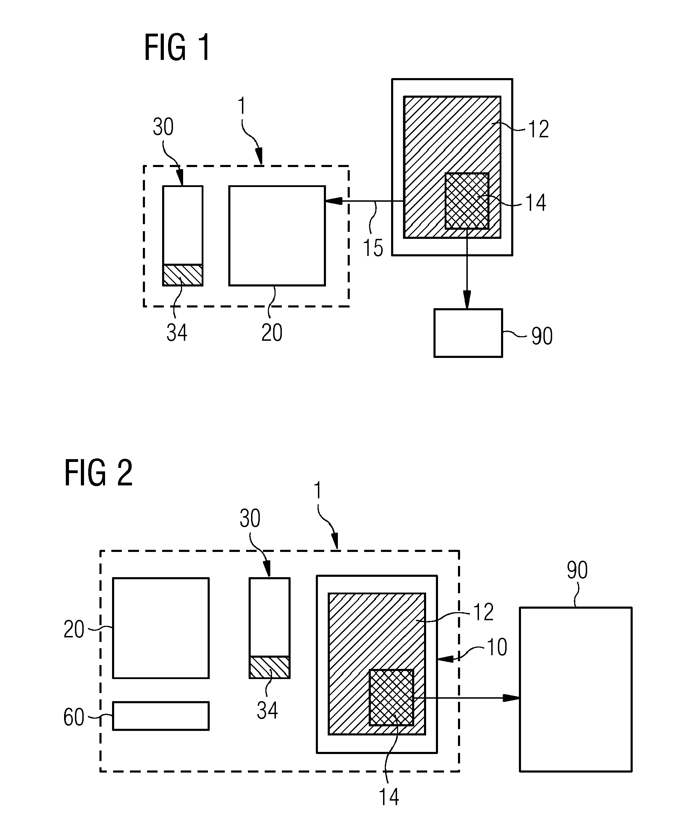

In FIG 1, an exemplary embodiment of a system 1 of the present technique is presented. The system 1 ensures authentication of an incoming touch-input received by a touch-sensitive surface 12. At least a region 14 on the touch-sensitive surface 12 activates a medical function of a medical device 90, or in other words the region 14 on the touch-sensitive surface 12 controls a functionality of the medical device 90. For example, the medical device 90 may be a X-Ray device and the region 14 may be a tab on the touch-sensitive surface 12, either physically separate from the X-ray device or integrated as a display in the X-Ray device, to direct the X-ray device to initiate a process of subjecting a patient or a subject to X-rays in order to acquire an image of a part of the subject. The system 1 includes a verification module 20 and an authentication unit 30.

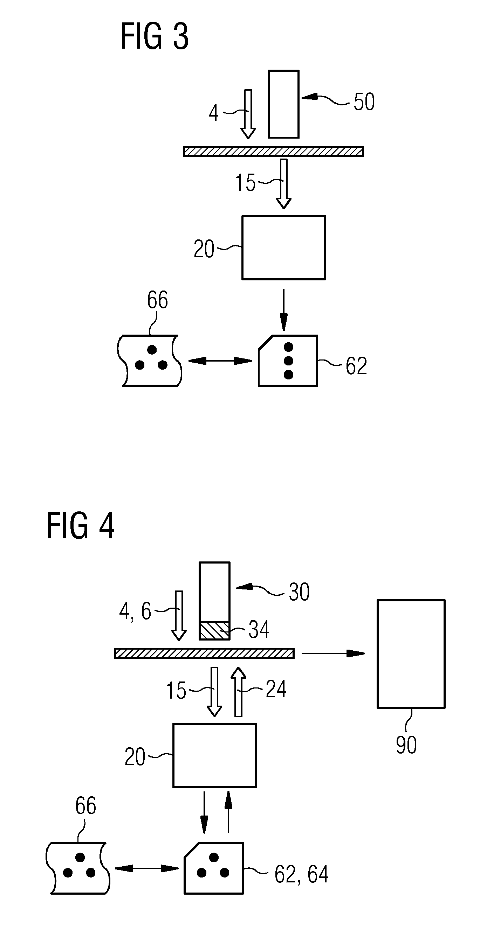

The system 1 has been explained hereinafter with reference to FIG 1 in combination with FIGs 3 and 4. FIG 3 schematically illustrates a functioning of the exemplary embodiment of the system 1 and FIG 4 schematically illustrates another functioning of the exemplary embodiment of the system 1. When an incoming touch-input 4, as depicted in FIGs 3 and 4, is received by the touch-sensitive surface 12, information 15 is provided by the touch-sensitive surface 12 to the verification module 20. The information 15 corresponds to the incoming touch-input 4. The verification module 20 receives the information 15 from the touch-sensitive surface 12. After receiving the information 15 the verification module 20 determine a received pattern 62 of the incoming touch-input 4 from the information 15.

The term 'pattern' and related terms, as used herein, means a discernible quality of the incoming touch-input 4 for example the number of pixels on the touch-sensitive surface 12 that were touched or contacted by the incoming touch-input 4, or whether the contact was a simple touch i.e. one point touch or a multi-touch, in case of multi-touch contact what was the number of touches and what was a relative orientation between these touches - the relative distances and positions with respect to each other, and so on and so forth. The 'pattern' may also be understood as geometric patterns or geometric arrangements of the different parts of the incoming touch-input 4 that give the incoming touch-input 4 a discernible quality. Furthermore, for example, a pattern formed by contacting the touch-sensitive surface 12 by a single finger tip is different from a pattern formed by contacting the touch-sensitive surface 12 by three finger tips simultaneously as part of a single incoming touch-input 4. For more example, a pattern formed by contacting the touch-sensitive surface 12 by three finger tips simultaneously as part of a single incoming touch-input 4 is different from a pattern formed by simultaneously contacting the touch-sensitive surface 12 by three metallic tips arranged in a fixed orientation with respect to each other as part of a same stylus.

In the system 1, the verification module 20 is further configured to compare the received pattern 62 to an authenticated pattern 66. The authenticated pattern 66 is a pattern, having the same meaning as explained hereinabove, that is predetermined and provided to the verification module 20 as a standard i.e. a pattern that is used for purposed to compare and determine the discernible quality of the received pattern 62. The comparing of the received pattern 62 to the authenticated pattern 66 performed by the verification module 20 may be done by a pattern matching algorithm. The verification module 20 may include, but not limited to, a processor. Such pattern matching algorithms are known and used pervasively in image processing and data matching techniques and thus has not been explained herein in greater details for sake of brevity.

The system 1, as shown in FIG 1, also includes an authentication unit 30. The authentication unit 30 is a physical entity having a particular form and structure. The authentication unit 30 includes a unique identity part 34 operable to provide a touch input 6 to the touch-sensitive surface 12. To be clearer, the incoming touch-input 4 provided to the touch-sensitive surface 12 by the unique identity part 34 is referred to as touch-input 6. The unique identity part 34 is designed in such a way that the touch input 6 provided by contacting the unique identity part 24 to the touch-sensitive surface 12 gives rise to a unique pattern 64 when identified by the verification module 20. To be clearer, when the unique identity part 34 is made to contact the touch-sensitive surface 12 by an operator (not shown) possessing and using the authentication unit 30, the received pattern 62 generated or extracted by the verification module 20, from the information 15 corresponding to the touch-input 6, is referred to as the unique pattern 64.

In accordance with aspects of the present technique, the unique pattern 64 is same as the authenticated pattern 66. In other words, the authenticated pattern 66 used by the verification module 20 is independently provided to or obtained by the verification module 20 but is a match of the unique pattern 64. It may also be said that the unique identity part 34 of the authentication unit 30 is designed in such a way that the pattern it provides on contacting the touch-sensitive surface 12 is a match of the authenticated pattern 66 which is pre-stored in the verification module 20 or in any other part of the system 1 for use by the verification module 20 while comparing the received pattern 62 with the authenticated pattern 66.

In the system 1, the verification module 20 is further configured to control the touch-sensitive surface 12 in such a way that the region 14 of the touch- sensitive surface 12 activates the medical function if and only if the received pattern 62 matches the authenticated pattern 66. In other words, the verification module 20 controls the touch-sensitive surface 12 in such a way that the region 14 of the touch- sensitive surface 12 activates the medical function if and only if the incoming touch-input 4 is provided to the touch-sensitive surface 12 by the unique identity part 34 of the authentication unit 30.

The control of the verification module 20 on the touch-sensitive surface 12 may be achieved either by using a signaling process between the verification module 20 and the touch-sensitive surface 12 directly or by using a signaling process between the verification module 20 and an intermediate controller (now shown) positioned logically between the touch-sensitive surface 12 and the medical device 90. For example, a micro-processor functioning as the intermediate controller may implement blocking of all commands communicated from the touch-sensitive surface 12 to the medical device 90 and may unblock one or more commands communicated from the touch-sensitive surface 12 to the medical device 90 only after receiving specific instructions from the verification module 20 to unblock. According to aspects of the present technique such specific instructions to unblock one or more commands communicated from the touch-sensitive surface 12 to the medical device 90 are provided by the verification module 20 to the intermediate controller if and when the received pattern 62 when compared by the verification module 20 to the authenticated pattern 66 is determined by the verification module 20 to be a match.

FIG 3 shows functioning of the system 1 when the incoming touch-input 4 in provided by contacting an unauthorized input device or unit 50 with the touch-sensitive surface 12. In accordance with aspects of the present technique, as a result of the contact of the touch-sensitive surface 12 by the unauthorized unit 50, the information 15 is received by the verification module 20 from the touch-sensitive surface 12. The information 15 is then analyzed by the verification module 20 and the received pattern 62 is extracted from the information 15 by the verification module 20. As depicted in FIG 3, the received pattern 62, for example, is a pattern of three dots linearly arranged. The verification module 20 then compares the received pattern 62 of FIG 3 with the authenticated pattern 66 of FIG 3 represented for example as three dots non-linearly arranged. As a result of the comparing, the verification module 20 determines that the received pattern 62 of FIG 3 does not match the authenticated pattern 66 of FIG 3, and thus the verification module 20 controls the touch-sensitive surface 12 in such a way that the medical function of the medical device 90 (not shown in FIG 3) is not activated.

FIG 4 schematically presents a scenario when the incoming touch-input 4 is provided by the authentication unit 30, or to be more precise, when the incoming touch-input 4 in provided by contacting the unique identity part 34 of the authentication unit 30 with the touch-sensitive surface 12. For ease of understanding, the incoming touch-input 4 is now termed as the touch-input 6 since it is provided by the unique identity part 34. In accordance with aspects of the present technique, as a result of the contact of the touch-sensitive surface 12 by the unique identity part 34, the information 15 is received by the verification module 20 from the touch-sensitive surface 12. The information 15 is then analyzed by the verification module 20 and the received pattern 62 is extracted from the information 15 by the verification module 20. For ease of understanding, the received pattern 62 is now termed as the unique pattern 64 since it is extracted from the touch-input 6 provided by the unique identity part 34. As depicted in FIG 4, the unique pattern 64, for example, is a pattern of three dots non-linearly arranged. The verification module 20 then compares the unique pattern 64 of FIG 4 with the authenticated pattern 66 of FIG 4, which is same as the authenticated pattern 66 of FIG 3 and represented for example as three dots non-linearly arranged. As a result of the comparing, the verification module 20 determines that the received pattern 62 of FIG 4 matches the authenticated pattern 66 of FIG 4, and thus the verification module 20 controls, for example by providing unblocking command 24, the touch-sensitive surface 12 in such a way that the medical function of the medical device 90 is activated.

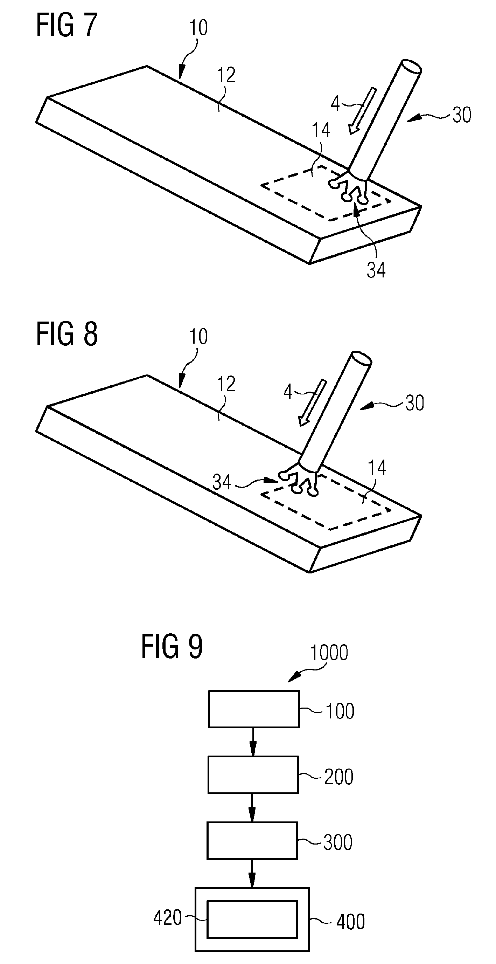

Now referring to FIGs 7 and 8, in combination with FIG 1, another exemplary embodiment of the system 1 and its functioning has been explained hereinafter. In this exemplary embodiment of the system 1, the verification module 20 controls the touch-sensitive surface 12 such that the region 14 of the touch-sensitive surface 12 activates the medical function only if the incoming touch-input 4 is confined entirely in the region 14 on the touch-sensitive surface 12. As shown in FIG 7, the unique identity part 34 of the authentication unit 30 has contacted the touch-sensitive surface 12 in such a way that the incoming touch-input is entirely confined with the region 14 on the touch-sensitive surface 12. In other words, as shown in FIG 7, the unique identity part 34 provides the touch input 6, giving rise to the unique pattern 64, by geographically limiting the physical contacting of the unique identity part 34 in the region 14 of the touch-sensitive surface 12. In such a scenario, the verification module 20 controls the touch-sensitive surface 12 such that the region 14 of the touch-sensitive surface 12 activates the medical function of the medical device 90.

Now referring to FIG 8, in combination with FIG 1 and as a comparison with FIG 7, the embodiment of the system 1 presented in FIG 7 has been further explained. As shown in FIG 8, the unique identity part 34 of the authentication unit 30 has contacted the touch-sensitive surface 12 in such a way that the incoming touch-input is not entirely confined with the region 14 on the touch-sensitive surface 12. In other words, as shown in FIG 8, the unique identity part 34 provides the touch input 6, giving rise to the unique pattern 64, but the physical contacting of the unique identity part 34 with the touch-sensitive surface 12 is not limited within the region 14. In such a scenario, the verification module 20 controls the touch-sensitive surface 12 such that the region 14 of the touch-sensitive surface 12 does not activate the medical function of the medical device 90.

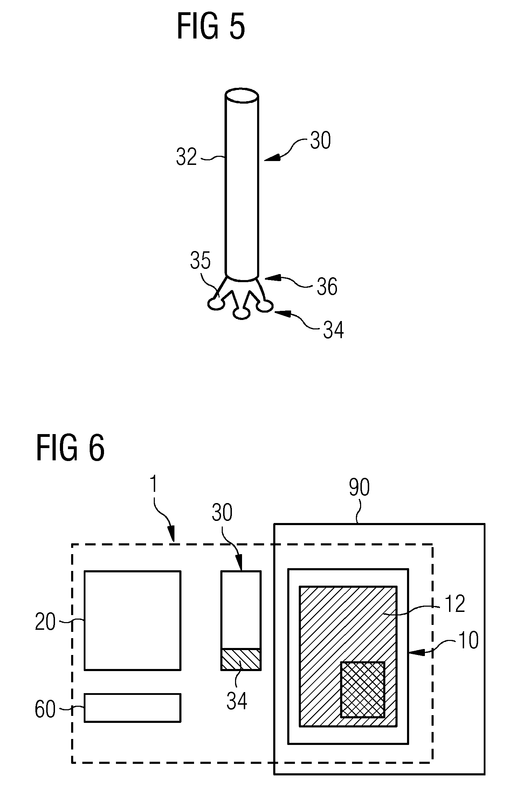

Referring now to FIG 2, another exemplary embodiment of the system 1 is presented. In this embodiment the system 1 includes a touch-screen unit 10 besides the verification module 20 and the authentication unit 30. The touch-screen unit 10 includes the touch-sensitive surface 12. In an exemplary embodiment of the system 1, as depicted in FIG 6, the touch-screen unit 10 is an integral part of the medical device 90 i.e. the touch-screen unit 10 is formed of a touch sensitive input such as touch enabled display (not shown) of the medical device 90. In an alternate embodiment of the system 1, the touch-screen unit 10 is remotely located with regard to the medical device 90, for example as depicted in FIG 2. When the touch-screen unit 10 is remotely located with regard to the medical device 90, the touch-screen unit 10 may be in wired, for example by data cable (not shown) or wireless communication, for example via Bluetooth, with the medical device 90. Thus the activation of the medical function of the medical device 90 from the touch-sensitive surface 12, or more particularly from the region 14 on the touch-sensitive surface 12 of the touch-screen unit 10 is communicated from the touch-screen unit 10 to the medical device 90 by connecting wires (not shown) or wirelessly.

Furthermore in another exemplary embodiment of the system 1, the verification module 20 and the touch-screen unit 10 are configured to communicate with each other wirelessly for example via Bluetooth technology and thus the information 15 from the touch-sensitive surface 12 of the touch-screen unit 10 is communicated to the verification module 20 wirelessly. Furthermore, any communication from the verification module 20 to the touch-sensitive surface 12 of the touch-screen unit 10, for example the unblocking command 24, is also communicated wirelessly. In an alternate embodiment (not shown) of the system 1, the verification module 20 and the touch-screen unit 10 are configured to communicate with each via wired connections for example via data cable and thus the information 15 from the touch-sensitive surface 12 of the touch-screen unit 10 is communicated to the verification module 20 transmitted through the data cable. Furthermore, any communication from the verification module 20 to the touch-sensitive surface 12 of the touch-screen unit 10, for example the unblocking command 24, is also communicated by transmitting through the data cable.

In an exemplary embodiment of the system 1, the touch-screen unit 10 is configured to control specific functions of the medical device 90 or may control a specific medical device 90 for example a C-arm based medical device 90 via the touch-sensitive surface 12.

Referring again to FIG 2, additional features of the system 1 are disclosed hereinafter. In an exemplary embodiment of the system 1, the system 1 includes a memory module 60. The memory module 60 stores the authenticated pattern 66. In this embodiment of the system 1, the verification module 20 receives or retrieves the authenticated pattern 66 from the memory module 60. The memory module 60 may store several different types of authenticated patterns 66 and the verification module 20 may retrieve a particular authenticated pattern 66 from the several different types of authenticated patterns 66 depending on the use of the system 1 for example depending on a type of authentication unit 30 used in the system 1.

It may be noted by one skilled in the art, that having a plurality of authentication unit 30 with different types of unique identity parts 34 giving rise to distinct corresponding unique patterns 64 is well within the scope of the present disclosure. In such an embodiment of the system 1 with a plurality of authentication unit 30 with different types of unique identity parts 34 giving rise to distinct corresponding unique patterns 64, the memory module 60 stores corresponding authenticated patterns 66 for each of the different types of unique identity parts 34. It may further be noted by one skilled in the art, that having a plurality of different types of unique identity parts 34 on a single authentication unit 30 such that each of the different type of unique identity part 34 gives rise to distinct corresponding unique patterns 64 is also well within the scope of the present disclosure. In such an embodiment of the system 1 with a single authentication unit 30 having a plurality of different types of unique identity parts 34 giving rise to distinct corresponding unique patterns 64, the memory module 60 stores corresponding authenticated patterns 66 for each of the different types of unique identity parts 34.

It may also be noted by one skilled in the art that the authentication unit 30 may be designed in various forms. For example, as depicted in FIG 5, the authentication unit 30 may be designed having an elongated body 32 for example as a stylus. The unique identity part 34 is located at one end 36 of the elongated body 32. The unique identity part 34 may be fashioned as a plurality of mechanical structures 35 positioned in a predefined relation or orientation to each other. The mechanical structures 35 of the unique identity part 34 are configured to be contacted simultaneously with the touch-sensitive surface 12, so as to give rise to the unique pattern 64 as aforementioned. The plurality of mechanical structures 35 may be fabricated as at least three protrusions 35 extending outward from the one end 36 of the elongated body 32, as depicted in FIG 5.

Now referring to FIG 9, a flow chart representing an exemplary embodiment of a method 1000 of the present technique and in accordance with aspects of the present technique is presented. The method 1000 has been explained hereinafter with reference to FIG 9 in combination with FIGs 1 to 8. The method 1000 is for authentication of the incoming touch-input 4 received by the touch-sensitive surface 12. The region 14 on the touch-sensitive surface 12 controls activation of a medical function of the medical device 90. In the method 1000, in a step 100, the information 15 is received from the touch-sensitive surface 12. The information 15 corresponds to the incoming touch-input 4. The information 15 may be received by a processor same as the verification unit 20 as explained hereinabove. After the step 100, in a step 200, the received pattern 62 of the incoming touch-input 4 is determined from the information 15. Thereafter, in a step 300, the received pattern 62 is compared to the authenticated pattern 66. Finally in the method 1000, in a step 400, the medical function is activated via the region 14 if and only if the received pattern 62 matches the authenticated pattern 66.

In an exemplary embodiment of the method 1000, as depicted also in FIG 9, the step 400 of the method 1000 includes a step 420 wherein the medical function of the medical device is activated only if the incoming touch-input 4 is entirely confined in the region 14 on the touch-sensitive surface 12. Thus in this embodiment of the method 1000, the region 14 activates the medical function of the medical device if and only if the received pattern 62 matches the authenticated pattern 66 and the incoming touch-input 4 is received physically confined within the region 14 of the touch-sensitive surface 12.

In another embodiment of the method 1000, the processor, same as the verification module 20 explained in FIGs 1 to 8, receives the information 15 from the touch-sensitive surface 12 wirelessly in the step 100 of the method 1000. In another embodiment of the method 1000, in the step 300 of comparing the received pattern 62 to the authenticated pattern 66, the authenticated pattern 66 which was previously stored in the memory module 60 is received by the processor from the memory module 60.

While the present technique has been described in detail with reference to certain embodiments, it should be appreciated that the present technique is not limited to those precise embodiments. Rather, in view of the present disclosure which describes exemplary modes for practicing the invention, many modifications and variations would present themselves, to those skilled in the art without departing from the scope and spirit of this invention. The scope of the invention is, therefore, indicated by the following claims rather than by the foregoing description. All changes, modifications, and variations coming within the meaning and range of equivalency of the claims are to be considered within their scope.

## Claims

1. A system (1) for authentication of an incoming touch-input (4) received by a touch-sensitive surface (12), wherein at least a region (14) on the touch-sensitive surface (12) is adapted to activate a medical function of a medical device (90), the system (1) comprising:
- a verification module (20) configured to receive an information (15) from the touch-sensitive surface (12), wherein the information (15) corresponds to the incoming touch-input (4), and to determine a received pattern (62) of the incoming touch-input (4) from the information (15), and wherein the verification module (20) is further configured to compare the received pattern (62) to an authenticated pattern (66); and
- an authentication unit (30) comprising an unique identity part (34) configured to provide to the touch-sensitive surface (12) a touch input (6) as the incoming touch-input (4), wherein the touch input (6) is configured to provide a unique pattern (64) as the received pattern (62) when identified by the verification module (20), and wherein the unique pattern (64) is same as the authenticated pattern (66) ;
wherein the verification module (20) is further configured to control the touch-sensitive surface (12) such that the region (14) of the touch- sensitive surface (12) activates the medical function only if the received pattern (62) matches the authenticated pattern (66).

2. The system (1) according to claim 1, wherein the verification module (20) is further configured to control the touch-sensitive surface (12) such that the region (14) of the touch-sensitive surface (12) activates the medical function only if the incoming touch-input (4) is confined entirely in the region (14) on the touch-sensitive surface (12).

3. The system (1) according to claim 1 or 2, comprising a touch-screen unit (10) having the touch-sensitive surface (12).

4. The system (1) according to claim 3, wherein the touch-screen unit (10) is an integral part of the medical device (90).

5. The system (1) according to claim 3, wherein the touch-screen unit (10) is remotely located with regard to the medical device (90).

6. The system (1) according to claim 5, wherein the touch-screen unit (10) is configured to communicate with the medical device (90) via wireless communication.

7. The system (1) according to any of claims 3 to 6, wherein the verification module (20) and the touch-screen unit (10) are configured to communicate with each other wirelessly.

8. The system (1) according to any of claims 3 to 7 wherein the touch-screen unit (10) is configured to control a C-arm based medical device (90) via the touch-sensitive surface (12).

9. The system (1) according to any of claims 1 to 8 further comprising a memory module (60) configured to store the authenticated pattern (66) and wherein the verification module (20) is configured to receive the authenticated pattern (66) from the memory module (60).

10. The system (1) according to any of claims 1 to 9, wherein the authentication unit (30) comprises an elongated body (32), and wherein the unique identity part (34) is located at one end (36) of the elongated body (32), the unique identity part (34) comprising a plurality of mechanical structures (35) positioned in a predefined relation to each other, and wherein the mechanical structures (35) are configured to be contacted simultaneously with the touch-sensitive surface (12).

11. The system (1) according to claim 10, wherein the plurality of mechanical structures (35) are at least three protrusions (35) extending outward from the one end (36) of the elongated body (32).

12. A method (1000) for authentication of an incoming touch-input (4) received by a touch-sensitive surface (12), wherein at least a region (14) on the touch-sensitive surface (12) is adapted to activate a medical function of a medical device (90), the method (1000) comprising:
- receiving (100) an information (15) from the touch-sensitive surface (12), wherein the information (15) corresponds to the incoming touch-input (4);
- determining (200) a received pattern (62) of the incoming touch-input (4) from the information (15);
- comparing (300) the received pattern (62) to an authenticated pattern (66); and
- activating (400) the medical function via the region (14) only if the received pattern (62) matches the authenticated pattern (66).

13. The method (1000) according to claim 12, wherein activating (400) the medical function via the region (14) only if the received pattern (62) matches the authenticated pattern (66) includes activating (420) the medical function only if the incoming touch-input (4) is entirely confined in the region (14) on the touch-sensitive surface (12).

14. The method (1000) according to claim 12 or 13, wherein a processor receives the information (15) from the touch-sensitive surface (12) wirelessly, and wherein the processor determines the received pattern (62) of the incoming touch-input (4) from the information (15) and compares the received pattern (62) to the authenticated pattern (66).

15. The method (1000) according to claim 14, wherein the authenticated pattern (66) is stored in a memory module (60) and wherein the processor receives the authenticated pattern (66) from the memory module (60).
